# EUROPEAN PATENT APPLICATION

(11) **EP 4 607 226 A1**
(43) Date of publication of application: **27.08.2025**
(21) Application number: 24158885.4
(22) Date of filing: 21.02.2024
(51) Int. Cl.: G01R 33/54, G16H 50/20

(54) **LARGE LANGUAGE MODEL GENERATION OF TOKENIZED PULSE SEQUENCE DATA**

(71) Applicant: Koninklijke Philips N.V., 5656 AG Eindhoven (NL)
(72) Inventor: WANG, Xinyu, 5656AG Eindhoven (NL); AMTHOR, Thomas Erik, 5656AG Eindhoven (NL)
(74) Representative: Philips Intellectual Property & Standards

(57) **Abstract**

Disclosed herein is a medical system (100, 300, 500) comprising a memory (110) storing machine executable instructions (120) and a domain-specific large language model (122). The large language model is configured to output tokenized pulse sequence data (126) in response to receiving subject data (124). The tokenized pulse sequence data is configured as tokens chosen from a pulse sequence token library (322). The medical system further comprises a computational system (104). Execution of the machine executable instructions causes the computational system to receive (200) the tokenized pulse sequence data in response to inputting the subject data into the domain-specific large language model.

## Description

### FIELD OF THE INVENTION

The invention relates to magnetic resonance imaging, in particular to the automated generation of pulse sequence data for the configuration of magnetic resonance imaging systems.

### BACKGROUND OF THE INVENTION

A large static magnetic field is used by Magnetic Resonance Imaging (MRI) system or scanner to align the nuclear spins of atoms as part of the procedure for producing images within the body of a patient. This large static magnetic field is referred to as the B0 field or the main magnetic field. By controlling gradient magnetic fields and radio-frequency pulses the nuclear spins can be manipulated to produce radio-frequency signals that can be sampled or measured as k-space data. The k-space data can then be reconstructed into a magnetic resonance image which images internal anatomical structures of the subject. The time dependent control of the radio-frequency signals and radio-frequency pulses, as well as the sampling of the k-space data are described in pulse sequence settings. Properly configuring the magnetic resonance imaging system requires extensive training and experience.

### SUMMARY OF THE INVENTION

The invention provides for a medical system, a method, a computer program, a method of training a domain-specific large language model, and a domain-specific large language model in the independent claims. Embodiments are given in the dependent claims.

In one aspect a medical system is disclosed. The medical system comprises a memory storing machine-executable instructions and a domain-specific large language model. The domain-specific large language model is configured to output tokenized pulse sequence data in response to receiving subject data. The tokenized pulse sequence data is configured as tokens chosen from a pulse sequence token library. The medical system further comprises a computational system. Execution of the machine-executable instructions causes the computational system to receive the tokenized pulse sequence data in response to inputting the subject data into the domain-specific large language model.

In another aspect a method is disclosed. The method comprises receiving tokenized pulse sequence data in response to inputting subject data into a domain-specific large language model. The domain-specific large language model is configured to output tokenized pulse sequence data in response to receiving subject data. The tokenized pulse sequence data is configured as tokens chosen from a pulse sequence token library.

In another aspect a computer program is disclosed. The computer program comprises machine-executable instructions and a domain-specific large language model with both configured for execution by a computational system. The large language model is configured to output tokenized pulse sequence data in response to receiving subject data. Execution of the machine-executable instructions causes the computational system to receive the tokenized pulse sequence data in response to inputting the subject data into the large language model. The tokenized pulse sequence data is configured as tokens chosen from a pulse sequence token library.

In another aspect a method of training a domain-specific large language model is disclosed. The method comprises receiving a foundation large language model. The method further comprises providing a pre-trained large language model by training the foundation large language model with medical documents comprising pulse sequence command specifications. The providing of the pre-trained large language model comprises generating a preliminary tokenized pulse sequence library. The method further comprises receiving site-specific medical records. The site-specific medical records comprise subject medical data paired with historical pulse sequence commands. The method further comprises generating the pulse sequence token library by excluding portions of the preliminary tokenized pulse sequence library absent from the site-specific medical records. The method further comprises providing the domain-specific large language model by training the pre-trained large language model with the site-specific medical records. Training the pre-trained large language model comprises at least partially tokenizing the site-specific medical records with the pulse sequence token library.

In another aspect a domain-specific large language model trained according to the method of training the domain-specific large language model is disclosed.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates an example of a medical system.
Fig. 4 shows a flow chart which illustrates a method of using the medical system of Fig. 3.
Fig. 5 illustrates an example of a medical system.
Fig. 6 shows a flow chart which illustrates a method of using the medical system of Fig. 5.
Fig. 7 illustrates a method of using a domain-specific large language model.
Fig. 8 illustrates a method of training a domain-specific large language model.
Fig. 9 illustrates an example of a user interface.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example a medical system comprises a memory storing machine-executable instructions and a domain-specific large language model. A domain-specific large language model as used herein encompasses a large language model. The label domain-specific is to indicate that the large language model has been trained for a particular use.

A large language model (LLM) as used herein encompasses a neural network architecture, typically built up of transformers (encoders and decoders) with self-attention layers and residual connections, that is a language model that has been trained using unlabeled text using self-supervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

The domain-specific large language model is configured to output tokenized pulse sequence data in response to receiving subject data. Tokenized pulse sequence data encompasses pulse sequence parameters and/or specifications that are encoded in a numeric form. The subject data encompasses data or metadata which is descriptive of a subject. The tokenized pulse sequence data may be converted into a verbal description of a pulse sequence protocol or be converted directly into parameters used for configuring a set of pulse sequence commands for acquiring k-space data.

The tokenized pulse sequence data is configured as tokens chosen from a pulse sequence token library. The pulse sequence token library may be different in different examples. In once case the pulse sequence token library may contain identifiers for particular sequence protocols or for the individual parameters used to customize a pulse sequence. The domain-specific large language model has been configured to output tokenized pulse sequence data that is chosen from this pulse sequence token library. It therefore does not output arbitrary descriptions or pulse sequence parameters but is limited to those in the pulse sequence token library. This may for example be a means of controlling the output of the domain-specific large language model to match those of a particular clinical site or sites.

The medical system further comprises a computational system. Execution of the machine-executable instructions causes the computational system to receive the tokenized pulse sequence data in response to inputting the subject data into the domain-specific large language model. This example may be beneficial because it may provide for a means of generating tokenized pulse sequence data that is customized to particular subject data. The limitation of the tokenized pulse sequence data being selected from the pulse sequence token library may have the effect of forcing the domain-specific large language model to provide valid tokenized pulse sequence data that may be useful in a clinical setting.

In another example execution of the machine-executable instructions further causes the computational system to generate pulse sequence commands from the tokenized pulse sequence data using the pulse sequence token library. The pulse sequence commands are configured for controlling a magnetic resonance imaging system to acquire k-space data. This example may be beneficial because it may provide for a means of fully automating the operation of a magnetic resonance imaging system. It may for example be useful for generating pulse sequence commands automatically or may be used as an assistant to an operator of a magnetic resonance imaging system.

In another example, execution of the machine-executable instructions further causes the computational system to display the pulse sequence commands using a user interface. Execution of the machine-executable instructions further causes the computational system to receive pulse sequence command modification data in response to displaying the pulse sequence commands using the user interface. Execution of the machine-executable instructions further causes the computational system to modify the pulse sequence commands using the pulse sequence modification data. Execution of the machine-executable instructions further causes the computational system to train the domain-specific large language model using the subject data and the modified pulse sequence commands.

In this example there may be two things which are achieved. The first is to provide a means or assistance for generating the pulse sequence commands in a particular clinical setting. The reception of the pulse sequence command modification data may for example be useful in fine tuning or adjusting the pulse sequence commands. The pulse sequence command modification data may for example be received either from an operator or from an additional algorithm or machine learning component. The modified pulse sequence commands and the subject data may then also be fed into the domain-specific large language model to provide additional training. As the pulse sequence commands are updated, they are used to improve the performance of the domain-specific large language model in generating tokenized pulse sequence data.

In another example the medical system further comprises the magnetic resonance imaging system. Execution of the machine-executable instructions further causes the computational system to acquire the k-space data by controlling the magnetic resonance imaging system with the pulse sequence commands. This example may be beneficial because it may provide for a means of automating or assisting the acquisition of k-space data by a magnetic resonance imaging system.

In another example the medical system further comprises a pulse sequence template database storing pulse sequence templates that are each configured for generating pulse sequence commands. A pulse sequence template may for example be a pulse sequence for a particular magnetic resonance imaging protocol which has typical values inserted. In other examples the template may be an algorithm which is used to generate pulse sequence commands upon receiving values which specify the pulse sequence parameters. Execution of the machine-executable instructions further causes the computational system to receive a selected pulse sequence template by querying the pulse sequence template database with at least a portion of the tokenized pulse sequence data. This querying may be done in different ways in different examples. In one example the tokenized pulse sequence data may contain an identifier which is used to select the selected pulse sequence template. In other examples the tokenized pulse sequence data may have a particular format or sequence of commands specified in the tokenized pulse sequence database which is recognized and then used to select the selected pulse sequence template. Execution of the machine-executable instructions further causes the computational system to generate the pulse sequence commands by inputting the tokenized pulse sequence data into the selected pulse sequence template. This may include using the tokenized pulse sequence data to modify various pulse sequence parameters of the selected pulse sequence template.

In another example the domain-specific large language model is further configured to output chain-of-thought data descriptive of the tokenized pulse sequence data. The chain-of-thought data is data which is used to explain or provide information on why the large language model had a particular output. This is known in the art and has the benefit of improving the quality of the domain-specific large language model. It may also be used by a person or additional algorithm to evaluate multiple choices that are output by the domain-specific large language model. Execution of the machine-executable instructions further causes the computational system to receive the chain-of-thought data from the domain-specific large language model when receiving the tokenized pulse sequence data.

In another example the tokenized pulse sequence data specifies multiple alternative magnetic resonance imaging protocols. The domain-specific large language model is configured for providing the chain-of-thought data for each of the multiple alternative magnetic resonance imaging protocols. Execution of the machine-executable instructions further causes the computational system to provide translated pulse sequence data as text associated with respective multiple alternative magnetic resonance imaging protocols by translating the tokenized pulse sequence data using the pulse sequence token library. Execution of the machine-executable instructions further causes the computational system to provide the chain-of-thought data for the respective multiple alternative magnetic resonance imaging protocols.

Execution of the machine-executable instructions further causes the computational system to receive a selection of selected magnetic resonance imaging protocols in response to providing the translated pulse sequence data as text associated with the respective multiple alternative magnetic resonance imaging protocols and in response to providing the respective chain-of-thought data for each of the multiple alternative magnetic resonance imaging protocols. The pulse sequence commands are generated for the selected magnetic resonance imaging protocol. This example may be beneficial because it may provide for a means of selecting the selected magnetic resonance imaging protocol from a larger number of magnetic resonance imaging protocols generated by the domain-specific large language model. This, for example could be achieved by receiving the selection from a user interface or from another software component.

In another example the memory further comprises a decision module that is configured for providing the selection of the selected magnetic resonance imaging protocol in response to receiving the translated pulse sequence data and the chain-of-thought data. The execution of the machine-executable instructions further causes the computational system to receive the selected magnetic resonance imaging protocol in response to inputting the translated pulse sequence data and the chain-of-thought data into the decision module. This may be beneficial because it may provide a means of automatically selecting the selected magnetic resonance imaging protocol. The decision module could be implemented in different ways. For example, it may use an additional large language model to provide for this decision making process. The additional large language model could for example be trained by recording the selection of a human operator a large number of times and using this for training this additional large language model.

In another example the tokenized pulse sequence data comprises magnetic resonance imaging protocol identifiers, pulse sequence parameters, and combinations thereof. The magnetic resonance imaging protocol identifiers could for example be used to identify a pulse sequence commands for a particular magnetic resonance imaging protocol and/or anatomical region to be imaged. The pulse sequence parameters may for example be various parameters which are used to modify or customize the pulse sequence to a particular clinical situation.

In another example the pulse sequence parameters comprise any one of the following: a pulse sequence repetition time, an echo time, a specification of a field of view, a flip angle, a k-space sampling method or pattern, an inversion time, a pulse shape definition, for example for a radio-frequency pulse, a gradient waveform for a magnetic field gradient, and combinations thereof. This example may be beneficial because these various parameters may be useful in customizing a particular set of pulse sequence to a particular clinical situation.

In another example the subject data comprises any one of the following: a clinical query, a clinical referral, a subject medical record, a prior written medical report, a patient's current medical condition, a patient's current medical condition, patient data descriptive of ability to move and communicate, patient data descriptive of ability for performing breath holds, patient metadata, patient age, patient body mass index, patient age, patient gender, the spoken language of the patient, an intravenous access data, and combinations thereof.

In some examples the subject data may also contain anatomical information such as location of anatomical markers on the surface of a subject or in a survey scan.

In another example a computer program comprises machine-executable instructions and also comprises a domain-specific large language model that are both for execution by a computational system. The large language model is configured to output tokenized pulse sequence data in response to receiving subject data. Execution of the machine-executable instructions causes the computational system to receive the tokenized pulse sequence data in response to inputting the subject data into the large language model. The tokenized pulse sequence data is configured as tokens chosen from a pulse sequence token library.

In another example there is a method of training a domain-specific large language model. The method comprises receiving a foundation large language model. A foundation large language model as used herein encompasses a large language model that has been trained using general knowledge and data. The foundation large language model may for example be a widely available large language model such as the ChatGPT-4 or Alpaca models. The method further comprises providing a pre-trained large language model by training the foundation large language model with medical documents comprising pulse sequence command specifications. The pulse sequence command specifications are data which describe the structure of the pulse sequences explicitly. The medical documents may for example be journal articles which discuss particular pulse sequence commands, textbooks which describe the structure and function of pulse sequence commands, as well as historical records and data of prior used pulse sequence commands used in a clinical setting. The medical documents may for example comprise such things as a written description of the use of these various pulse sequence commands. The providing of the pre-trained large language model comprises generating a preliminary tokenized pulse sequence library.

The foundation large language models typically have a system for tokenizing text that is input into them. The tokenization may be based on assigning tokens for individual letters, word fragments or entire words. However, these foundation large language models do not have the specific tokenization for particular medical documents. The preliminary tokenized pulse sequence library may be an additional token library that is generated during the course of training the foundation large language model with the medical documents that comprise or contain the pulse sequence command specifications. The method of training the domain-specific large language model further comprises receiving site-specific medical records. These, for example may include medical records that are used or generated at a particular clinical site. The site-specific medical records comprise subject medical data paired with historical pulse sequence commands. For example, at a particular clinic or hospital there may be a collection of medical records which contain data about the pulse sequences used and the condition of the subject paired with these pulse sequence data.

The method further comprises generating the pulse sequence token library by excluding portions of the preliminary tokenized pulse sequence library absent from the site-specific medical records. This for example could be achieved by using the preliminary token pulse sequence library to tokenize the site-specific medical records. Tokens which were not selected during this tokenization process may then be excluded from the resulting token library. This may have the effect of preventing preliminary tokens in the preliminary token pulse sequence library from being used when they are not present in the site-specific medical records. The method further comprises providing the domain-specific large language model by training the pre-trained large language model with the site-specific medical records. The training the pre-trained large language model comprises at least partially tokenizing the site-specific medical records with the pulse sequence token library. This example may have the benefit of providing a domain-specific large language model that is adapted for generating tokenized pulse sequence data that is customized for a particular clinical site and may be used for configuring particular magnetic resonance imaging systems.

In another example there is a domain-specific large language model trained according to the method of training the domain-specific large language model.

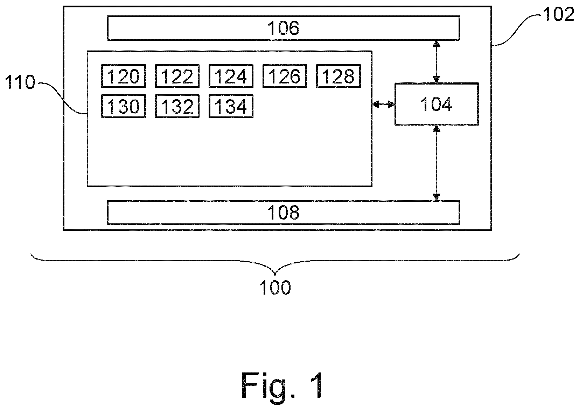

Fig. 1 illustrates an example of a medical system 100. The medical system 100 is shown as comprising a computer 102. The computer 102 may represent one or more computers 102 located at one or more locations. The computer 102 comprises a computational system 104. The computational system 104 may represent one or more computational systems or computational cores that are located at one or more locations also. The computer 102 is further shown as containing an optional hardware interface 106 and an optional user interface 108 which are both in communication with the computational system 104. The hardware interface 106 may enable the computational system 104 to control other components of the medical system 100 if they are present. It may also enable the exchange of data with a network or other components of the medical system 100. The user interface 108 may enable an operator or user to control the operation and function of the medical system 100. The medical system 100 is further shown as comprising a memory 110. The memory 110 is intended to represent various types of memory which may be accessible to the computational system 104. In one example the memory 110 is a non-transitory storage medium.

The memory 110 is shown as containing machine-executable instructions 120. The machine-executable instructions 120 may enable the computational system 104 to perform various computational and data manipulation and image processing tasks. The machine-executable instructions 120 may for example also be used or enable the computational system 104 to control other components of the medical system 100 via the optional hardware interface 106. The memory 110 is further shown as containing a domain-specific large language model 122. The domain-specific large language model is configured for outputting tokenized pulse sequence data 126 in response to receiving subject data 124. The tokenized pulse sequence data 126 may be restricted to tokens that are present in a pulse sequence token library. The subject data 124 may for example be received via a network connection or from another database. In some examples the subject data 124 may be partially received from the user interface 108. The tokenized pulse sequence data 126 is shown as being stored in the memory 110 and was received from the domain-specific large language model 122 when the subject data 124 was input.

The memory 110 is further shown as containing an optional pulse sequence template database 128. The pulse sequence template database 128 contains templates which may be used to provide example or fillable algorithms which generate pulse sequences according to a particular or chosen magnetic resonance imaging protocol. The memory 110 is further shown as containing a database query 130. The database query 130 may for example be reconstructed or constructed from the tokenized pulse sequence data 126. In some examples the tokenized pulse sequence data 126 may contain an identifier that is directly used as the database query 130. In other examples a fingerprint or sequence of commands found in the tokenized pulse sequence data 126 are used to construct the database query 130. The database query 130 is used to query the pulse sequence template database 128 and in response a selected pulse sequence template 132 is received.

The tokenized pulse sequence data 126 is then used to modify the selected pulse sequence template 132 and produce the optional pulse sequence commands 134. These pulse sequence commands 134 may be used to control a magnetic resonance imaging system to acquire k-space. With these optional features the medical system 100 comprises a control system which may be used to automatically configure a magnetic resonance imaging system to acquire k-space data using simply the subject data 124.

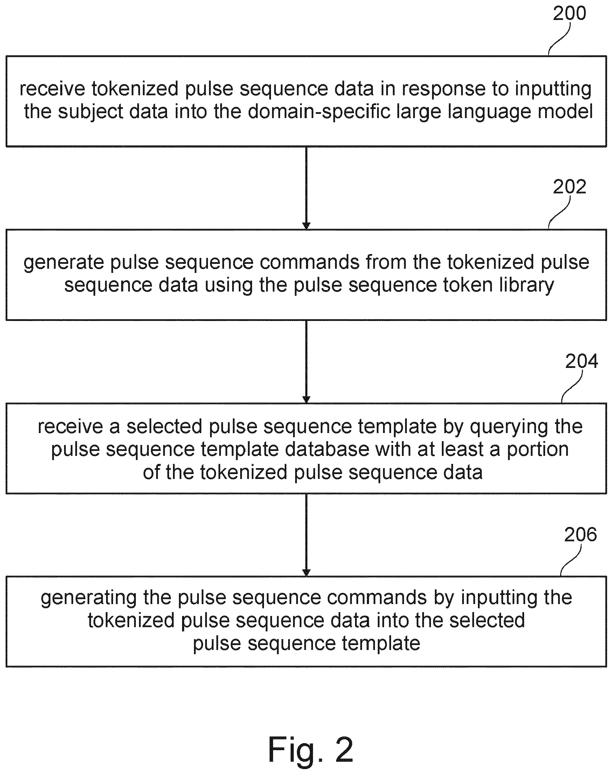

Fig. 2 shows a flowchart which illustrates a method of operating the medical system 100 in Fig. 1. In step 200 the tokenized pulse sequence data 126 is received in response to inputting the subject data 124 into the domain-specific large language model 122. Steps 202, 204, and 206 are optional. In step 202 the pulse sequence commands 134 are generated from the tokenized pulse sequence data 126 using the pulse sequence token library. For example, the pulse sequence token library is used to provide particular pulse sequence parameters from individual tokens that are present in the tokenized pulse sequence data. In this particular example this generation of the pulse sequence is indirect. Step 202 may be performed by doing steps 204 and 206 as illustrated. In step 204 the selected pulse sequence template 132 is received by querying 130 the pulse sequence template database 128 with at least a portion of the tokenized pulse sequence data 126.

In step 206 the pulse sequence commands 134 are generated by inputting the tokenized pulse sequence data 126 into the selected pulse sequence template 132. In some cases, this may involve first converting the tokenized pulse sequence data 126 into individual data or numerical data which is then used to modify the selected pulse sequence template 132. In other examples the selected pulse sequence template 132 may be an algorithm which is configured to receive the tokenized pulse sequence data 126 directly. For example, it may be a fillable form that upon receiving particular tokens the selected pulse sequence template is automatically modified or generates the pulse sequence commands.

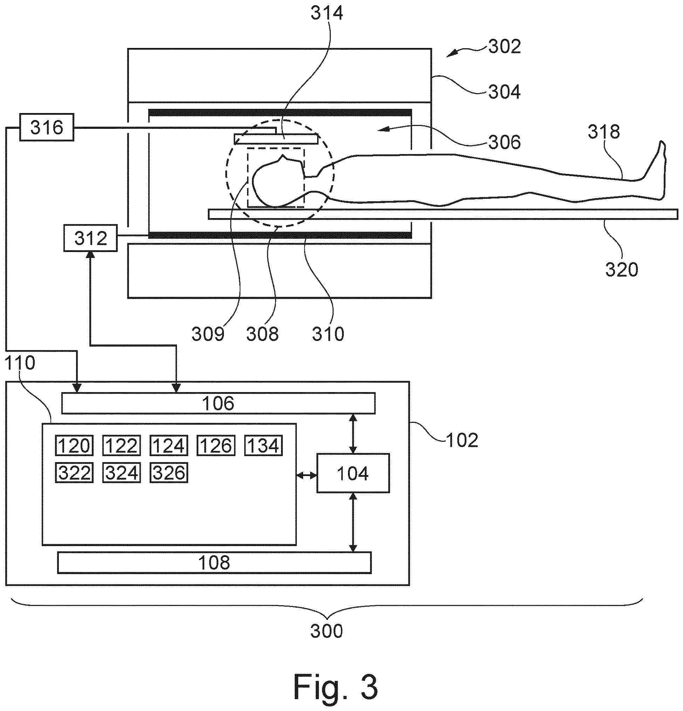

Fig. 3 illustrates a further example of a medical system 300. The medical system 300 in Fig. 3 is similar to the medical system 100 in Fig. 1 except that it additionally comprises a magnetic resonance imaging system 302. The magnetic resonance imaging system 302 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The measured k-space data is acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309. In this example, the head of the subject 318 is positioned within the field of view 309. The measured k-space data that will be acquired is therefore for performing a head or brain scan.

Within the bore 306 of the magnet there is also a set of magnetic field gradient coils 310 which is used for the acquisition of measured k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory is further shown as containing a pulse sequence token library 322. In this instance the pulse sequence token library 322 is used to convert the tokenized pulse sequence data 126 into the pulse sequence commands 134. In some examples the method illustrated in Fig. 2 may be used. The memory 110 is further shown as containing k-space data 324 that was acquired by controlling the magnetic resonance imaging system with the pulse sequence commands 134. The memory 110 is further shown as containing a magnetic resonance image 326 that was reconstructed from the k-space data 324.

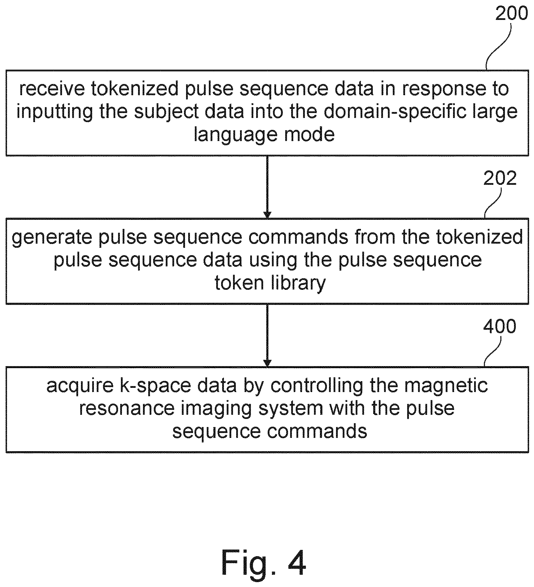

Fig. 4 shows a flowchart which illustrates a method of operating the medical system 300 of Fig. 3. Steps 200 and 202 are performed as was illustrated in Fig. 2. In step 400 the k-space data 324 is acquired by controlling the magnetic resonance imaging system 302 with the pulse sequence commands 134. Although it is not shown in this diagram the method may be continued by reconstructing the magnetic resonance image 326 from the k-space data 324. In some instances, the features of Figs. 1 and 3 may be combined. Likewise, the steps illustrated in Fig. 2 may also be combined with the steps illustrated in Fig. 4.

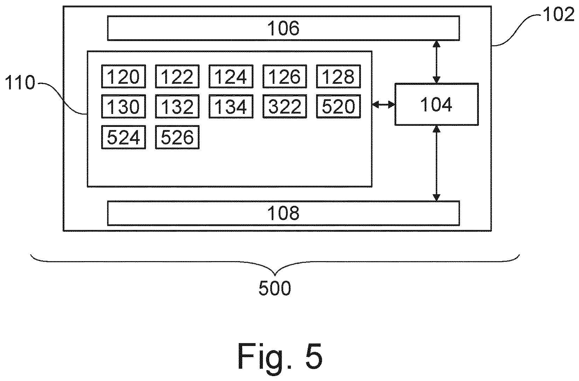

Fig. 5 illustrates a further example of a medical system 500. In this example the domain-specific large language model 122 is additionally configured to output chain-of-thought data 520 at the same time that it is outputting the tokenized pulse sequence data 126. Additionally in this example the tokenized pulse sequence data 126 is output for multiple separate pulse sequence protocols. In this example the domain-specific large language model 122 outputs several different suggestions and provides the chain-of-thought data 520 for each one. This chain-of-thought data 520 may be used to help select the selected magnetic resonance imaging protocol 524. In this example the pulse sequence token library 322 is used to generate a translated pulse sequence data 522 from the tokenized pulse sequence data 126. The chain-of-thought data 520 and the translated pulse sequence data 522 is provided for each suggestion individually and be used to select a selected magnetic resonance imaging protocol 524.

The selected magnetic resonance imaging protocol 524 then has its respective tokenized pulse sequence data 126 used to generate the pulse sequence commands 134. In some examples this data may be presented on a user interface and a user may then choose the selected magnetic resonance imaging protocol 524. This process may also be automated. The memory 110 is further shown as containing an optional decision module 526 that receives the chain-of-thought data 520 and the translated pulse sequence data 522 for each suggestion and then outputs the selected magnetic resonance imaging protocol 524. The optional decision module 526 may for example be implemented using an additional large language model that has been configured for this purpose.

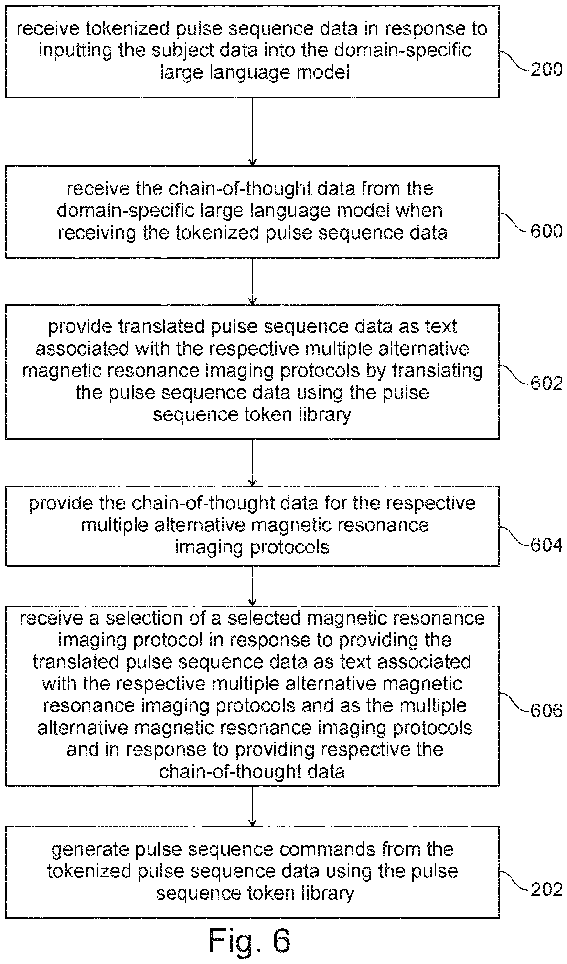

Fig. 6 shows a flowchart which illustrates a method of operating the medical system 500 of Fig. 5. Step 200 is performed as was illustrated in Figs. 2 and 4. In step 600 the chain-of-thought data 520 is received from the domain-specific large language model 122 when the tokenized pulse sequence data 126 was received. In this case the tokenized pulse sequence data 126 contained several different alternatives. In step 602 the translated pulse sequence data 522 is provided as text with the respective multiple alternative magnetic resonance imaging protocols by translating the tokenized pulse sequence data that was tokenized using the pulse sequence token library 322. In step 604 the chain-of-thought data 520 for the respective multiple alternative magnetic resonance imaging protocols is provided. In step 606 the selection of the selected magnetic resonance imaging protocol 524 is received in response to providing the translated pulse sequence data 522 and the respective chain-of-thought data 520. Next in step 202 the pulse sequence commands 134 are generated from the tokenized pulse sequence data, in this case the portion corresponding to the selected magnetic resonance imaging protocol 524 and the pulse sequence token library 322.

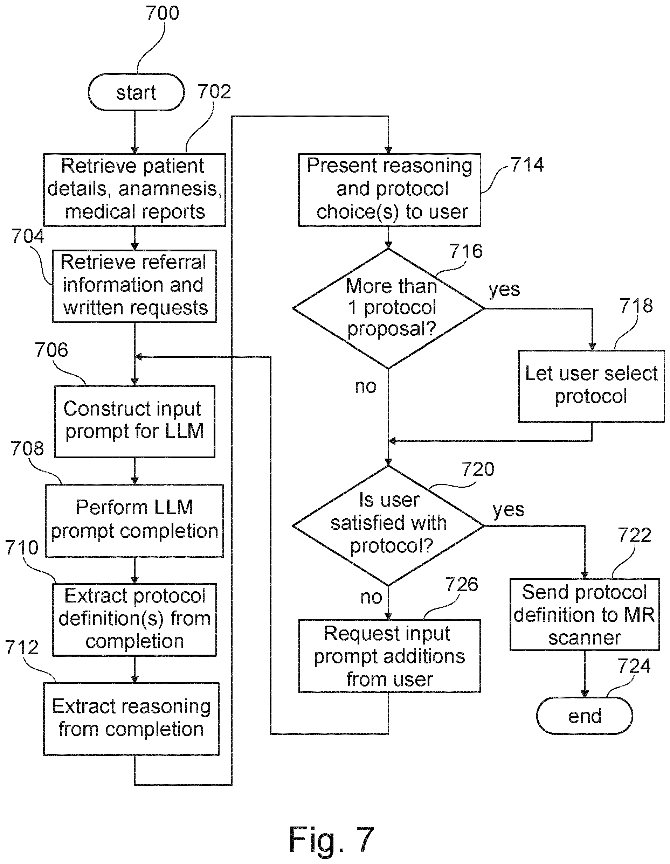

Fig. 7 illustrates a supplementary method that may be performed in conjunction with the methods illustrated in Figs. 2, 4, and 6. At step 700 the method starts. In step 702 various subject data 124 is retrieved. This may for example include patient details, an anamnesis, and medical reports. In step 704 additional subject data 124 is retrieved. This may include additional referral information and written requests. In step 706 the input prompt for the domain-specific large language model 122 is constructed. In step 708 the prompt completion for the domain-specific large language model 122 is performed. In step 710 the protocol definitions for the tokenized pulse sequence data 126 is extracted. In step 712 the chain-of thought data 520 is extracted from the output of the domain-specific large language model 122 also. In step 714 the translated pulse sequence data 522 and chain-of-thought data 520 for each of the multiple suggestions are presented to the operator.

Step 716 is a decision box and has more than one protocol suggested in the translated pulse sequence data 522. If the answer is "yes" then in step 718 either a user or the decision module 526 selects a particular magnetic resonance imaging protocol 524. In box 716 if the answer is "no" then the method proceeds to block 720. After block 718 is performed the method arrives at block 720 also. In step 720 the user is prompted if the user is satisfied with the selected magnetic resonance imaging protocol 524. If the answer is yes the method proceeds to step 722 where the protocol definition is sent to the magnetic resonance imaging system 302. This may include generating the pulse sequence commands 134. In block 720, if the user is not satisfied with the selected magnetic resonance imaging protocol 524 the system may prompt the operator for additional data from the user. This may for example be used to add additional data to add to the prompt for the domain-specific large language model 122. The method then goes back to step 706.

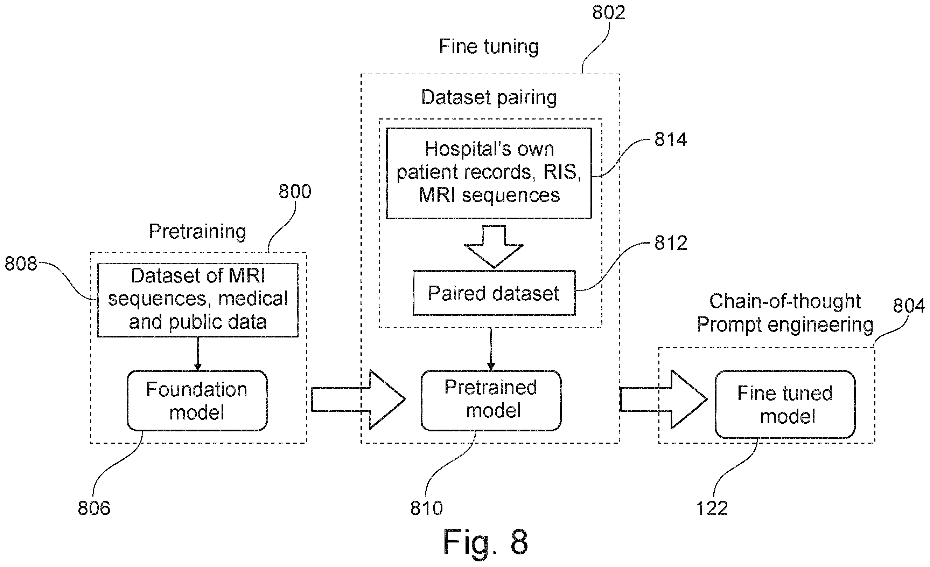

Fig. 8 shows a flowchart which illustrates one method of training the domain-specific large language model 122. This may be divided into three parts. There is a pretraining part 800, a fine tuning part 802, and optional chain-of-thought data prompt engineering 804.

In the pretraining 800 the foundation model 806 is received and this is trained with medical documents 808. This provides a pre-trained model 810. The medical documents 808 may include, for example, a combination of Magnetic Resonance Imaging (MRI) sequences, variable settings, protocol names, medical and public data. The pretraining 808 may start with preprocessing the input by tokenizing the textual input into machine understandable numbers. While tokenization is not new for conventional textual data, tokenizing MRI sequences while maintaining its clinical significance is new. One approach is to segment each sequence name by separators (e.g., hyphen or underscore), then apply 1 to N-gram tokenization. For example, sequence name as 'T2W_SSH_Cor_trigg' would result in 7 tokens: `T2W', `SSH', 'Cor', 'trigg', `T2W SSH', `T2W SSH Cor' and `T2W SSH Cortrigg'. A more sophisticated approach is to add the variables and their values of the sequences into consideration, e.g., TR (repetition time), TE (echo time), field of view, flip angle and more. Despite differences in the naming of protocols across MRI producers, the setting of scanning variables identifies a sequence, and the order of the sequences defines an MRI protocol. By tokenizing the pre-formatted variable and value pairs while memorizing the mapping of protocol names to sequences and their settings, the preprocessing step shall integrate the MRI sequence language to the readable clinical information. The output of this step is a set of tokens, i.e., a mixture of vocabularies.

Next, depending on the usage frequency in the input, a weighting scheme could be applied to assign numbers to all the tokens, resulting in a matrix with all the tokens as columns and their normalized weights as values. This matrix may then be used as the input to the foundation model, whose initial parameters get updated during training, e.g., via backpropagation. The output of the pretraining is a set of parameters with optimized values. The pertained model 810 may have learned the semantic relations of tokens, their ordering, and structures, adapted to medical domain regarding MRI protocols. For example, in the semantic space of the tokens, sequence term Magnetic resonance cholangiopancreatography or MRCP would be close to abdomen or Liver Metastasis.

In the fine tuning part 802, the hospital's own patient records and magnetic resonance imaging sequences 814 are used to generate site-specific medical records 812. It contains data about individual patients containing patient data and magnetic resonance imaging protocols. This is then used to train the pre-trained model into the fine tuned model 122.

Targeting the task of `translating clinical input into a list of MRI sequences', fine tuning may use the pre-trained model and specifically structured data input to instruct the pre-trained model to solve the targeted task. Depending on user's needs, this module could also adapt to address multiple tasks, e.g., summarization of clinical input, then recommendation of MRI protocol. Input data for this module shall be limited to a certain clinical entity, e.g., one specific hospital, so the fined tuned model could give the most suitable output that best adapt the hospital's own language usage.

For the translation task, this module could use a sub-module of data pairing, which takes hospital's own clinical data and MRI protocols (i.e., protocol names, the list of sequences, and the variable-value pairs of each sequence), and converts them into pairs, e.g., in the form of (original text, MRI protocol). The pairs shall be then used as input to tune the pre-trained model. During the fine-tuning process, the values of parameters of the pre-trained model would get further updated to suit the translation task, so that when given a piece of clinical information combined with patient info and diagnostic purpose, a list of MRI sequences would be generated.

To align the output of the fine-tuned model to human reference, the model may be continuously updated by human feedback, e.g., based on human user giving a positive or negative response to a recommended MRI protocol, the model updates its parameter values to adapt its alignment to human reference. For this purpose, a reward model could be utilized to penalize or reward model, to trigger or automate the adaptation.

Once the fine tuned model 122 is prepared it may optionally be refined using chain-of-thought data prompt engineering 804. During the training of the foundation model 806 the medical documents 808 may be tokenized into a preliminary tokenized pulse sequence library. In generation of the paired dataset 812 there may be a tokenizing step and it may be noted which tokens of the preliminary token pulse sequence library were not used. These tokens may then be excluded and from this exclusion be used to generate the pulse sequence token library. During the training of the pre-trained model 810 into the fine tuned model 122 only the pulse sequence token library 322 is used.

After fine turning the model, a process of prompt engineering with chain-of-thought mechanism could be further established to improve model's output. This step is to instruct the model to output not only a list of MRI sequences, but also its reason. This may use a number of instructions in the form of `clinical information -> reason -> MRI protocol' as input to the fine-tuned model. In this example, the method in this example may be adapted for clinical radiology scenarios, focusing on the specific task of recommending MRI protocol.

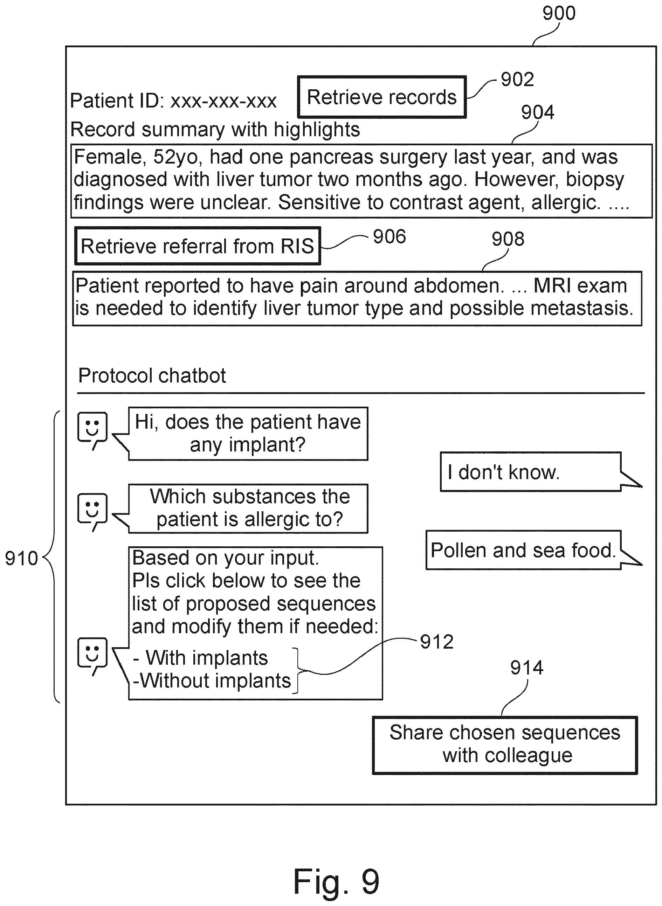

In some examples, the fine-tuned model 122 may upgrade itself via continuous human feedback. This could be realized by an interactive application. Fig. 9 below illustrates a user interface of a possible application. Examples may be able to capture user's input, address it, and output options with over-threshold likelihood. In case of unsatisfied recommendation, the model model 122 adjust according to user's input, and output an improved answer. Besides, it shall be able to adapt the changes or missing of input, based on which it outputs selectable answers to the users. Via the interaction with users, the backend model shall be able to further align with human preference but maintain some degree of generalization, in order not to overfit human input.

An example prompt (data input into the the domain-specific large language module 122) may be:

*We know the following details about the patient to be examined:*
*<patient details from RIS>*

*The referring physician has requested to following:*
*<referral details and reports>*

*Please propose a suitable protocol definition for the required MRI exam. Give a step-by-step reasoning for your proposal.*

An example of the completion of the prompt (the output of domain-specific large language model 122):
*For this case, I know that the patient does not suffer from claustrophobia, and from the patient details and anamnesis I infer that the patient will be able to lie still and follow instructions easily. Based on the referral, it is obvious that both an anatomical image and a physiological image are required. I therefore propose the following protocol:* (example of train-of-though data)
*[protocol start]*
   *Survey*
   *T1W axial*
   *T2W axial*
   *DWI*
*[prototol end]* (example of MR protocols selected)

Several concrete examples are presented below:
In a first example, the example prompt is used as input to the domain-specific large language model and a selection of MR protocols to perform are output. In this case the operation of the MR system is not automated, but instead provides assistance to the operator. As in the above example, the prompt (subject data 124) input to the domain-specific large language model 122 is:
*We know the following details about the patient to be examined:*
*<patient details from RIS>*

*The referring physician has requested to following:*
*<referral details and reports>*

*Please propose a suitable protocol definition for the required MRI exam. Give a step-by-step reasoning for your proposal.*

The resulting output of the domain-specific large language model 122 is then:
*For this case, I know that the patient does not suffer from claustrophobia, and from the patient details and anamnesis I infer that the patient will be able to lie still and follow instructions easily. Based on the referral, it is obvious that both an anatomical image and a physiological image are required. I therefore propose the following protocol:* (example of train-of-though data)
*[protocol start]*
   *A1*
   *A2*
   *A3*
   *A4*
*[prototol end]* (example of MR protocols selected)

The first portion of this output is the chain-of-thought data and the values A1, A2, A3, and A4 are tokens representing a selection of individual MR acquisitions to be performed: Survey, T1W axial, T2W axial, DWI. The pulse sequence token library 322 in this case could be used to covert these values A1, A2, A3, and A4 to the text description Survey, T1W axial, T2W axial, DWI. Alternatively these values could be used to retrieve a template for a pulse sequence or could be used to trigger a preprogrammed configuration of the magnetic resonance imaging system.

In a second example, the tokenized pulse sequence template data provides more detailed information and may be used to configure the magnetic resonance imaging system automatically. In this example the input prompt (subject data) 124 could comprise:
*Subject metadata (subject height, weight, sex)*
*Identification of anatomy to be imaged*
*Request from physician for particular clinical test or*
*Geometrical location of subject with respect to magnetic resonance imaging system, this could, for example, be provided by fiducial markers on the subject, an identification of location by a anatomical key point locator using a camera, or via a survey scan with anatomical landmarks identified. The Geometrical location registers the location of the subject to the MR system.*
*Possibly prior electronic medical records.*

*Please propose a pulse sequence for the required MRI exam. Give a step-by-step reasoning for your proposal.*

An example output would for example be:
*Based on the subject data input and the requirement of the physician a Diffusion Weighted Image (DWI) is proposed. Tokens which select and specify the appropriate pulse sequence are detailed below.*

### A3 B7 4E AE 41 28 98 E1

The values A3 B7 4E AE 41 28 98 E1 are a code, with A3 being used to query a pulse sequence template database 128 for the Diffusion Weighted Image pulse sequence. In this case a template of a premade DWI pulse sequence is retrieved. The values B7 4E AE 41 28 98 E1 are then used to customize or modify the pulse sequence to the particular acquisition with: B7 specifying a Slice Selection, B7 4E AE specifying the field of view, 41 specifying a configuration of the RF pulses, 28 specifying a configuration of the Diffusion Gradient pulses, 98 specifying an echo time (TE), and E1 specifying a Pulse Repetition time. In this example the details output by the domain-specific large language model 122 are quite detailed and can be used to configure the MR system. The domain-specific large language model 122 is able to perform this because it was trained with the site-specific medical records 812 as illustrated in Fig. 8. The historical subject data and prior performed MR pulse sequences contained in the site-specific medical records 814 is used to prepare the site specific medical records 812 and provides this detailed information during training.

Fig. 9 illustrates an example of an interactive user interface that may be used to interact with the medical system illustrated in Figs. 1, 3, or 5. When asking for additional user input, the system can act as a chatbot. For this application, the domain-specific large language model may have received additional training to interact with the user in the case of an unclear protocol definition to ask for exactly the required details to make a better decision about a protocol. The figure also includes the example of a suggestion of two different protocols from which the user may choose.

In this example, the user interface 900 comprises a button 902 to retrieve medical records which may be equivalent to retrieving the subject data 124. There is an additional button to retrieve information from the RIS 906 with an additional text input box 908, where additional data descriptive of the subject may be input. The medical system may also use a chatbot and provide a protocol chatbot 910 to ask additional questions from the operator. Instead of typing in text directly the large language model may be configured to ask questions from the medical practitioner to provide at least a portion of the subject data 124. The chatbot 912 may also have a region where the operator is able to select 912 between different protocols. The links labeled 912 functions as a selector for a magnetic resonance imaging protocol. Once a protocol has been selected there may also be an additional share button 914 that enables the operator to share the choice of a medical protocol with colleagues.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: domain-specific large language model
- 124: subject data
- 126: tokenized pulse sequence data
- 128: pulse sequence template database
- 130: database query
- 132: selected pulse sequence template
- 134: pulse sequence commands
- 200: receive tokenized pulse sequence data in response to inputting the subject data into the domain-specific large language model
- 202: generate pulse sequence commands from the tokenized pulse sequence data using the pulse sequence token library
- 204: receive a selected pulse sequence template by querying the pulse sequence template database with at least a portion of the tokenized pulse sequence data
- 206: generating the pulse sequence commands by inputting the tokenized pulse sequence data into the selected pulse sequence template
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio frequency coil
- 316: transceiver
- 318: subject
- 320: subject support
- 322: pulse sequence token library
- 324: k-space data
- 326: magnetic resonance image
- 400: acquire k-space data by controlling the magnetic resonance imaging system with the pulse sequence commands
- 500: medical system
- 520: chain-of-thought data
- 522: translated pulse sequence data
- 524: selected magnetic resonance imaging protocol
- 526: decision module
- 600: receive the chain-of-thought data from the domain-specific large language model when receiving the tokenized pulse sequence data
- 602: provide translated pulse sequence data as text associated with the respective multiple alternative magnetic resonance imaging protocols by translating the tokenized pulse sequence data using the pulse sequence token library
- 604: provide the chain-of-thought data for the respective multiple alternative magnetic resonance imaging protocols
- 606: receive a selection of a selected magnetic resonance imaging protocol in response to providing the translated pulse sequence data as text associated with the respective multiple alternative magnetic resonance imaging protocols and as the multiple alternative magnetic resonance imaging protocols and in response to providing respective the chain-of thought data, wherein the pulse sequence commands are generated for the selected magnetic resonance imaging protocol
- 700: start
- 702: retrieve patient details, anamnesis, medical reports
- 704: retrieve referral information and written requests
- 706: construct input prompt for domain-specific large language model
- 708: perform domain-specific large language prompt completion
- 710: extract tokenized pulse sequence data from output of domain-specific large language model
- 712: extract chain-of-though data from output of domain-specific large language model
- 714: present reasoning (chain-of-though data) and protocol choices constructed from tokenized pulse sequence data
- 716: More than one protocol proposal?
- 718: Let user or algorithm select protocol
- 720: Is user satisfied with protocol?
- 722: Send protocol definition to magnetic resonance imaging scanner
- 724: end
- 726: request input prompt additions from user
- 800: pretraining portion
- 802: fine tuning portion
- 804: optional chain-of-though prompt engineering portion
- 806: foundation large language model
- 808: medical documents
- 810: pretrained model
- 812: site specific medical records
- 814: hospital's own patient records and MRI sequences
- 816: domain specific large language model
- 900: interactive API
- 902: button to retrieve medical records
- 904: record summary with highlights
- 906: button to retrieve Radiological Information System (RIS) data
- 908: additional text input area
- 910: protocol chatbot
- 912: selector for protocol
- 914: share button

## Claims

1. A medical system (100, 300, 500) comprising:
- a memory (110) storing machine executable instructions (120) and a domain-specific large language model (122), wherein the large language model is configured to output tokenized pulse sequence data (126) in response to receiving subject data (124), wherein the tokenized pulse sequence data is configured as tokens chosen from a pulse sequence token library (322); and
- a computational system (104), wherein execution of the machine executable instructions causes the computational system to receive (200) the tokenized pulse sequence data in response to inputting the subject data into the domain-specific large language model.

2. The medical system of claim 1, wherein execution of the machine executable instructions further causes the computational system to generate (202) pulse sequence commands (134) from the tokenized pulse sequence data using the pulse sequence token library, wherein the pulse sequence commands are configured for controlling a magnetic resonance imaging system (302) to acquire k-space data (324).

3. The medical system of claim 2, wherein execution of the machine executable instructions further causes the computational system to:
- display the pulse sequence commands using a user interface;
- receive pulse sequence command modification data in response to displaying the pulse sequence commands using the user interface;
- modify the pulse sequence commands using the pulse sequence modification data; and
- train the domain-specific large language model using the subject data and the modified pulse sequence commands.

4. The medical system of claim 2 or 3, wherein the medical system further comprises the magnetic resonance imaging system, wherein execution of the machine executable instructions further causes the computational system to acquire (400) the k-space data by controlling the magnetic resonance imaging system with the pulse sequence commands.

5. The medical system of claim 2, 3, or 4, wherein the medical system further comprises a pulse sequence template database (128) storing pulse sequence templates configured for generating pulse sequence commands, wherein execution of the machine executable instructions further causes the computational system to:
- receive (204) a selected pulse sequence template by querying the pulse sequence template database with at least a portion of the tokenized pulse sequence data; and
- generating (206) the pulse sequence commands by modifying the selected pulse sequence template with the tokenized pulse sequence data.

6. The medical system of any one claims 2 through 5, wherein the domain-specific large langue model is further configured to output chain-of-thought data (520) descriptive of the tokenized pulse sequence data, wherein execution of the machine executable instructions further causes the computational system to receive (600) the chain-of-thought data from the domain-specific large language model when receiving the tokenized pulse sequence data.

7. The medical system of claim 6, wherein the tokenized pulse sequence data specifies multiple alternative magnetic resonance imaging protocols, wherein the domain-specific large language model is configured for providing the chain-of-thought data for each of the multiple alternative magnetic resonance imaging protocols, wherein execution of the machine executable instructions further causes the computational system to:
- provide (602) translated pulse sequence data (522) as text associated with the respective multiple alternative magnetic resonance imaging protocols by translating the tokenized pulse sequence data using the pulse sequence token library;
- provide (604) the chain-of-thought data for the respective multiple alternative magnetic resonance imaging protocols; and
- receive (606) a selection of a selected magnetic resonance imaging protocol (524) in response to providing the translated pulse sequence data as text associated with the respective multiple alternative magnetic resonance imaging protocols and in response to providing the respective chain-of-thought data, wherein the pulse sequence commands are generated for the selected magnetic resonance imaging protocol.

8. The medical system of claim 7, wherein the memory further comprises a decision module (526) configured for providing the selection of the selected magnetic resonance imaging protocol in response to receiving the translated pulse sequence data and the chain-of-thought data, wherein execution of the machine executable instructions further causes the computational system to receive the selected magnetic resonance imaging protocol in response to inputting the translated pulse sequence data and the chain-of thought data into the decision module.

9. The medical system of any one of the following, wherein the tokenized pulse sequence data comprises any one of the following: magnetic resonance imaging protocol identifiers, pulse sequence parameters, and combinations thereof.

10. The medical system of claim 9, wherein the pulse sequence parameters comprise any one of the following: a pulse sequence repetition time, an echo time, a specification of a field of view, a flip angle, a k-space sampling method, an inversion time, a pulse shape definition, a gradient waveform, and combinations thereof.

11. The medical system of any one of the preceding claims wherein the subject data comprises any one of the following: a clinical query, a clinical referral, a subject medical record, a prior written medical report, patient's current medical condition, patient's mental condition, patient data descriptive of ability to move and communicate, patient data descriptive of ability for performing breathholds, patient metadata, patient age, patient body mass index, patient age, patient gender, spoken langue, IV access data and combinations thereof.

12. A method comprising receiving (200) tokenized pulse sequence data in response to inputting subject data (124) into a domain-specific large language model (122), wherein the domain-specific large language model is configured to output tokenized pulse sequence data in response to receiving subject data, wherein the tokenized pulse sequence data is configured as tokens chosen from a pulse sequence token library.

13. A computer program comprising machine executable instructions (120) and a domain specific large language model (122) for execution by a computational system (104), wherein the large language model is configured to output tokenized pulse sequence data (126) in response to receiving subject data, wherein execution of the machine executable instructions causes the computational system to receive the tokenized pulse sequence data in response to inputting the subject data into the domain-specific large language model, wherein the tokenized pulse sequence data is configured as tokens chosen from a pulse sequence token library (322).

14. A method of training a domain-specific large language model (122),
wherein the method comprises:
- receiving a foundation large language model (806);
- providing a pretrained large language model (810) by training the foundation large langue model with medical documents (808) comprising pulse sequence command specifications, wherein providing the pretrained large langue model comprises generating a preliminary tokenized pulse sequence library;
- receiving site specific medical records (812), wherein the site specific medical records comprise subject medical data paired with historical pulse sequence commands;
- generating a pulse sequence token library (322) by excluding portions of the preliminary tokenized pulse sequence library absent from the site specific medical records;
- providing the domain-specific large language model (122) by training the pretrained large language model with the site-specific medical records, wherein training the pretrained large language model comprises at least partially tokenizing the site-specific medical records with the pulse sequence token library.

15. A domain-specific large language model (122) trained according to claim 14.
